# EUROPEAN PATENT APPLICATION

(11) **EP 0 934 753 A2**
(43) Date of publication of application: **11.08.1999**
(21) Application number: 99300427.4
(22) Date of filing: 21.01.1999
(51) Int. Cl.: A61M 5/307

(54) **Needleless medical device for delivering of terapeutic agent**

(30) Priority: 05.02.1998 GB 9802506
(71) Applicant: The BOC Group plc, Windlesham Surrey GU20 6HJ (GB)
(72) Inventor: Garrett, Michael Ernest, Woking, Surrey, GU22 7XR (GB)
(74) Representative: Gough, Peter

(57) **Abstract**

A needleless syringe 1 comprises a hollow casing 2 having an outlet 4 and containing a first compartment 6 in which is located a fluid eg. helium capsule 8 and a second compartment 10 for a therapeutic agent. The compartments 6, 10 are separated by a partition 12 on which is mounted a needle 14 with a sharpened tip 16 which sits in a cavity 26 formed in the base 24 of the capsule 8. Activation of a fluid release mechanism 17 causes the capsule 8 to move towards the needle 14 which pierces the base 24 and releases the fluid from the capsule 8.

## Description

The present invention relates to needleless devices for delivering a therapeutic agent against the skin of a patient.

Such devices are known which harness the energy of a high pressure gas contained within a capsule at a pressure between 60 and 80 bar. Needleless devices avoid piercing the skin of a patient by utilising said high pressure gas for example, when a capsule is ruptured, to pass powdered medication or other substances through the skin of the patient. Needleless devices reduce the discomfort of puncturing the epidermis with a hypodermic needle which is of great comfort to patients with a needle phobia.

In PCT published application WO94/24263 there is described a needleless syringe which includes a metal capsule containing helium gas at high pressure which is used to force particles of a therapeutic agent through the skin of a patient in a substantially painless manner. The capsule is detachable from the remainder of the syringe and includes a hollow body for containing the helium under pressure and a neck extending from the hollow body which incorporates a spring loaded valve.

In use, a gas release device located at the proximal end of the syringe employs a cam to engage the base of the hollow body of the capsule thereby forcing the neck of the capsule against a hollow projection. The hollow projection effectively engages the spring loaded valve to open said valve to release the helium from the hollow body of the capsule. Once used, the capsule can either be recharged with gas or more favourably the capsule can be discarded and a new capsule already charged with gas can be attached to the syringe.

In the circumstances where the gas capsule is a throw away item it is important that it can be manufactured both simply and cheaply. A particular disadvantage of the gas capsule employed with the needleless syringe described in PCT published application W094/24263 is that by the incorporation of a spring loaded valve this increases the difficulty of manufacture and the filling of the capsule with gas with a consequent increase in costs.

In medical applications, helium is a favoured gas since it is very light which makes it suitable for use as a propellant for therapeutic agents in that when it impinges against the skin of a patient it will bounce off into the atmosphere and not pass through the skin of the patient. However, helium because it is light is difficult to contain since it will leak through the most minuscule fault in a capsule.

Furthermore, in medical applications it is important that the helium gas can be released from the gas capsule with a minimal force by the user for example finger force.

In European patent publication no. 0757200 there is described a needleless syringe which includes a capsule for storing a propellant fluid, for example, helium under pressure. The capsule is provided with a stopper having the stem formed with a frangible section. In use mechanical means is employed to rupture the stem about the frangible section to release helium under high pressure between 60 and 80 bar.

This arrangement where the stem is provided with a frangible section does offer the disadvantage that the stem can be completely detached from the remainder of the capsule and be forced by the escaping helium towards the patient. This necessitates the use of a filter between the gas capsule and the outlet of the syringe for catching any metal particles entrained by the helium. The use of a filter in this manner will of course absorb some of the force of the helium.

It is an aim of the present invention to obviate the disadvantages mentioned above with regard to the needleless medical devices known from the prior art.

It is an aim of the present invention to provide in a device for delivering a therapeutic agent against the skin of a patient, a capsule for fluid under high pressure which can be manufactured simply and cheaply.

It is a further aim to provide a capsule for fluid under pressure for example, helium gas which is substantially leak proof.

It is yet a further aim to provide a capsule which can be opened with finger pressure.

According to the present invention, a device for delivering a therapeutic agent against the skin of a patient comprises a hollow body open at a distal end to define an outlet; a first compartment in the hollow body for containing a capsule in which is stored a fluid under pressure; a second compartment in the hollow body located between the first compartment and the outlet for containing the therapeutic agent; a member having a sharpened portion and a fluid release mechanism mounted on the hollow body for moving the capsule and the member relatively towards each other thereby causing the sharpened portion of the member to pierce the capsule and allow the escape of fluid from the capsule and hence from the first compartment towards the second compartment for entraining the therapeutic agent, in which the member is mounted on a partition separating the first and second compartments, the partition being formed with a plurality of holes to permit the passage of fluid from the first towards the second compartment

In a preferred embodiment the sharpened portion of the member is initially located in a cavity formed in the base of the capsule.

Preferably, the member is in the form of a hollow needle with a sharpened distal tip. The needle is dimensional to seal within the cavity formed in the base of the capsule so that on release of the fluid from the capsule, said fluid avoids contact with the outside of the capsule. This arrangement avoids the needle for the capsule to be cleaned to stringent medical standards.

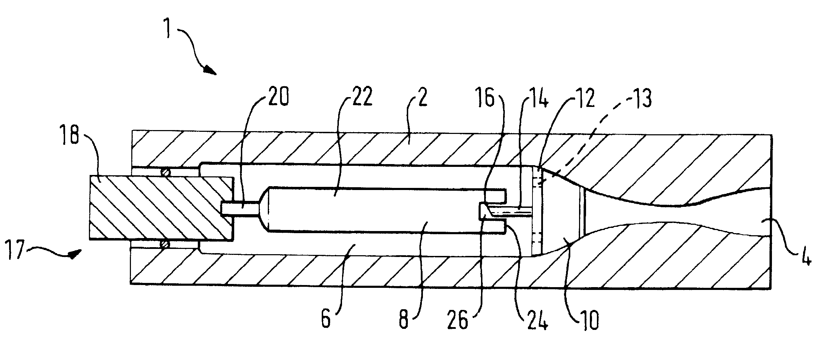

An embodiment of the invention will now be described by way of example, reference being made to the Figure of the accompanying diagrammatic drawing which is a longitudinal cross-section through a device for delivery a therapeutic agent against the skin of the patient.

As shown, a needleless medical device in the form of a needleless syringe 1 comprises essentially a casing 2 in the form of an elongate hollow tube open at a distal end to define an outlet 4. The casing 2 includes a first compartment 6 containing a capsule 8 and a second compartment 10 located between the first compartment 6 and the outlet 4 for containing a powdered therapeutic agent, for example, a drug. Separating the compartments 6, 10 is a partition 12 which supports a member 14 with a sharpened tip 16 at its free end. The partition 12 is formed with a plurality holes 13 which allow the passage of gas therethrough from compartment 6 towards compartment 10.

At its proximal end, the syringe 1 is provided with a fluid release mechanism which includes a movable button 18 slidably mounted in a gas tight manner within the casing 2. A portion of the button 18 extends, prior to use, beyond the proximal end of the casing 2 which portion can be engaged by a finger of a user of the syringe 1.

At its right hand (as shown) end, the button 18 is formed with a bore which receives the end of a stem 20 extending from a hollow body 22 of the capsule 8. The stem 20 which is hollow is utilised during the filling of the hollow body 22 with, for example, helium at between 60 and 80 bar.

The capsule 8 also includes a base 24 which is formed with a recess or cavity 26 which, as shown, receives the sharpened tip 16 of the member 14.

In use, when it is desire to treat a patient by means of the syringe 1, the drug is placed in the chamber 10 and the outlet 4 is placed against the skin of the patient.

Finger pressure is then applied to that end of the button 18 which extends beyond the distal end of the casing 2. This pressure causes the button to move towards the right (as shown) into the casing 2, carrying with it the capsule 8. This movement of the capsule 8 relative to the member 14 will cause the sharpened tip 16 of the

member 14 to pierce the base 24 of the capsule 8 with the subsequent release of helium. The released helium passes out from the compartment 6 through the holes 13 in the partition 12 and into the compartment 10 where it entrains the powdered drug. The helium with the entrained powdered drug then passes through the outlet 4 with the drug passing through the skin of the patient whilst the light molecules of helium bounce off the skin into the atmosphere.

In order to prevent any small metal particles which might be generated during the piercing of the capsule 8 from becoming entrained with the helium and being delivered to the patient, a filter may be disposed either between the base 24 of the capsule 8 and the partition 12 or in the compartment 10 immediately adjacent the partition 12.

In a modification, the member 14 may be in the form of a hollow needle with a sharpened distal tip, which needle is dimensioned to seal within the recess 26. This arrangement will avoid the fluid when released from the capsule 8 having any contact with the outer surface of the capsule and thereby reduce the need for the capsule to be medically clean.

Although the fluid release mechanism 17 has been shown with a sliding button 18 the button could be screw threaded so that rotation of the button 18 causes

longitudinal movement relative to the casing 2 with the same effect of moving the capsule 8 against the sharpened tip 16 of the member 14.

A safety device (not shown) may be incorporated in the fluid release mechanism to prevent premature or accidental actuation of the button 18.

A particular advantage of the above described embodiment is that the capsule contains no complicated valving arrangement and is therefore relatively simple to manufacture.

Further, by avoiding the use of a frangible section in the stem 20, said stem 20 is more robust and therefore the capsule is easier to handle and fill with fluid during the manufacturing process.

## Claims

1. A device (1) for delivering a therapeutic agent against the skin of a patient comprising a hollow casing (2) open at a distal end to define an outlet (4), a first compartment (6) in the hollow casing (2) containing a capsule (8) in which is stored a fluid under pressure, a second compartment (10) in the hollow casing (2) locate between the first compartment (6) and the outlet (4) for containing the therapeutic agent, a member (14) having a sharpened portion, and a fluid release mechanism (17) mounted on the hollow casing (2) for moving the capsule (8) and the member (14) relatively towards each other thereby causing the sharpened portion of the member (14) to pierce the capsule (8) and allow the escape of fluid from the capsule and hence from the first compartment (6) towards the second compartment (10) for entraining the therapeutic agent therein, in which the member (14) is mounted on a partition (12) separating the first and second compartments, the partition (12) being formed with a plurality of holes (13) to permit the passage of fluid from the first towards the second compartment.

2. A device as claimed in claim 1, in which, prior to use of the device, the sharpened portion of the member (14) in the form of a tip (16) is received in a cavity (26) formed in a base (24) of the capsule (8).

3. A device as claimed in claim 2, in which the member (14) is in the form of a hollow needle with a sharpened distal tip (16), said needle being dimensioned to seal within the cavity (26).

4. A device as claimed in any one claims 1 to 3, in which the fluid release mechanism (17) is located adjacent the proximal end of the hollow casing (2) and includes a movable button (18) partially extending from the proximal end of the hollow casing, the arrangement being such that pressure on the proximal surface of the button (18) causes the button to slide within the hollow casing (2) thereby causing the capsule (8) to move towards the sharpened portion of the member (14).

5. A device as claimed in any one of claims 1 to 3 in which the fluid release mechanism (17) is located adjacent the proximal end of the hollow casing (2) and includes a rotatable button partially extending from the proximal end of the hollow casing, the arrangement being such that by turning the button said button is caused to move within the hollow casing thereby causing the capsule to move towards the sharpened portion of the member.

6. A device as claimed in claim 4 or 5, in which the capsule (8) includes a hollow body (22) for storing the fluid under pressure and a stem (20) extending from said hollow body, at least a portion of the stem being received within a bore of the button.

7. A device as claimed in any one of claims 1 to 6 in which a safety mechanism is provided to prevent accidental actuation of the fluid release mechanism.

8. A device as claimed in any one of claims 1 to 7 in which a filter is located between the capsule (8) and the therapeutic agent when located in the second compartment (10).
